# EUROPEAN PATENT SPECIFICATION

(11) **EP 4 051 214 B1**
(45) Date of publication and mention of the grant of the patent: **18.11.2026**
(21) Application number: 20883293.1
(22) Date of filing: 30.10.2020
(51) Int. Cl.: A61K 8/02, A61F 13/00, A61K 9/70, A61M 35/00, A61P 17/02, D01D 5/00

(54) **METHOD FOR TREATING SKIN**
VERFAHREN ZUR BEHANDLUNG DER HAUT
MÉTHODE DE TRAITEMENT DE LA PEAU

(30) Priority: 31.10.2019 US 201962928734 P; 28.10.2020 US 202017082522
(43) Date of publication of application: 07.09.2022
(73) Proprietor: Kao Corporation, Chuo-Ku Tokyo 103-8210 (JP)
(72) Inventor: STRUEWING, Sharon, Cincinnati, Ohio 45214-1729 (US); HACHIYA, Akira, Cincinnati, Ohio 45214-1729 (US)
(74) Representative: Hoffmann Eitle
(86) International application number: PCT/US2020/058178
(87) International publication number: WO 2021/087239

(56) References cited:
- WO-A1-01/26610
- WO-A1-2018/097202
- WO-A1-2018/194132
- WO-A1-2018/196044
- US-A1- 2018 296 591
- US-A1- 2018 296 591
- US-A1- 2018 317 627
- RINA RAPHAEL: "This spray-on nanofiber 'skin' may revolutionize wound care", FASTCOMPANY.COM, 6 July 2019 (2019-07-06), pages 1 - 12, XP055932153, Retrieved from the Internet <URL:https://www.fastcompany.com/90365782/this-spray-on-nanofiber-skin-may-revolutionize-burn-and-wound-care>

## Description

### Field of the Invention

The present invention relates to a method for treating skin for improving various skin conditions occurring during skin epidermis treatment, such as chemical peeling.

### Background of the Invention

Cosmetic or dermatological treatment has been widely carried out, for example, to make spots, freckles, and wrinkles less conspicuous, and to perform color treatment on the skin, by performing chemical or physical treatment, such as chemical peeling, microdermabrasion, microneedling, laser treatment, tattoo treatment, etc. on the epidermis of the skin.

However, these treatments, even in the epidermis of the skin, can cause chemical or physical stimulus not only to the horny layer, but also to the granular layer, spinous layer and/or basal layer. For this reason, skin conditions such as inflammation, redness, and swelling often occur for 10 days to 4 weeks after the treatments. It has been pointed out that those who receive such treatment are hesitant to go out in public until the stimulated skin conditions are significantly improved. WO 2018/196044 A1 concerns a skin whitening program method comprising an external application step, a treatment step and an oral administration step.
WO 2018/194132 A1 relates to a method for forming a coating film, said coating film containing fibers, through electrostatic spray directly to the skin. US 2018/317627 A1 describes a film forming device including a distance measurement unit, a distance decision unit and a distance notification unit.
WO 2018/097202 A1 relates to a method for producing a coating film for cosmetic preparations including a cosmetic-preparati9on application step and an electrostatic spraying step.
US 2018/0296591 A1 describes methods including applying to the skin a composition comprising a reactive reinforcing component and a cross-linking component.

### Summary of the Invention

The present invention is defined by the appended claims and provides a composition X for use in a method for alleviating inflammation, redness and/or swelling of skin in a subject who has undergone a cosmetic or dermatological treatment,
wherein composition X comprises the following components (a) and (b):
   a) one or more volatile substances selected from the group consisting of water, an alcohol, and a ketone; and
   b) a polymer having an ability to form a film containing a fiber deposit; and
the method for alleviating inflammation, redness or swelling of skin comprises performing the following step A) and then step B):
   A) physically or chemically treating an epidermis of the skin; and
   B) forming a film containing a fiber deposit on the skin which has been subjected to the step A) with the composition X, wherein the step of forming a film containing a fiber deposit is performed by:
      (B1) electrostatically spraying the composition X on the skin or
      (B2) obtaining a film from the composition X by electrospinning or meltblowing on a surface, wherein the film is composed of a fiber deposit having an average fiber diameter of from 0.01 to 7 µm, and attaching said film to the skin.

Further, the present invention relates to Composition Z for use in a method for alleviating inflammation, redness and/or swelling of skin in a subject who has undergone a cosmetic or dermatological treatment,
wherein composition Z comprises the following components (c) and (d):
   (c) one or more selected from the group consisting of an oil agent and a polyol which are in a liquid state at 20°C; and
   (d) a fiber having an average fiber diameter ranging from 0.01 to 7 µm and an aspect ratio (average fiber length/average fiber diameter) ranging from 10 to 1,000; and
   the method for alleviating inflammation, redness or swelling of skin comprises performing the following step A) and then step B):
      A) physically or chemically treating an epidermis of the skin, wherein the treatment results in inflammation, redness or swelling of the skin; and
      B) forming a film containing a fiber deposit on the skin which has been subjected to the step A) by applying the composition Z to the skin

### Brief Description of the Drawings

FIG. 1 is a schematic diagram showing the structure of an electrostatic spray device suitably used in the present invention; and
FIG. 2 is a schematic diagram showing a situation in which an electrostatic spray method is carried out using the electrostatic spray device.

### Detailed Description of the Invention

For skin conditions after the above cosmetic or dermatological treatments, application of bactericides or anti-inflammatory agents and like treatments are performed; however, it is said that about 10 days to 4 weeks are required to improve the conditions.

Therefore, the present inventors conducted various studies on the means for quickly improving skin conditions after the aforementioned cosmetic or dermatological treatments, and then found that when the skin after cosmetic or dermatological treatment (step A)) was treated by forming a film containing a fiber deposit (step B)), conditions such as inflammation, redness, and swelling were alleviated, and these downtimes disappeared more quickly. Thus, the present invention has been completed.

According to the present invention, when the skin is treated with the conventional step A), followed by forming a film containing a fiber deposit (step B)), conditions such as inflammation, redness, and swelling are alleviated, these conditions disappear more quickly, and the quality of life (QOL) of persons who have undergone the cosmetic or dermatological treatment is significantly improved.

The present invention relates to a composition X or Z for use in a method for alleviating inflammation, redness and/or swelling of skin in a subject who has undergone a cosmetic or dermatological treatment, the method comprising performing the following step A) and then step B):
A) physically or chemically treating an epidermis of the skin; and
B) forming a film containing a fiber deposit on the skin which has been subjected to the step A).

A means for applying a film made of nanofibers obtained by electrospinning to a wound site, a means for using the film as a cosmetic sheet, and a means for directly applying the film to healthy skin are known (JP-A-2005-290610, JP-A-2008-179629, WO 2001/12139, etc.). However, it is not known at all whether the film can be applied to skin with physically or chemically treated epidermis, as in the step A) mentioned above, and what effect will appear when the film is applied to such skin.

The step A) is a step of physically or chemically treating the epidermis of the skin.

Here, the epidermis of the skin refers to the surface of the skin, the horny layer, the granular layer, the prickle layer, and the basal layer. Cellular components of the epidermis include a small amount of melanocytes, Langerhans cells, and Merkel cells, in addition to horny cells, granular cells, spinous cells, and basal cells, as well as.

The step A) is preferably a step of physically or chemically treating one or more selected from the group consisting of, among these epidermal layers, the horny layer, the granular layer, the spinous layer, and the basal layer. Since the treatment of these layers is highly likely to cause conditions such as inflammation, the step A) is more preferably a step of physically or chemically treating one or more selected from the group consisting of the granular layer, the spinous layer, and the basal layer. The present invention includes epidermis treatments extending to the papillary dermis.

Examples of the means for physically or chemically treating the epidermis include a means for physically stimulating cells present in the epidermis, a means for chemically treating cells present in the epidermis, and a means for using physical stimulation and chemical treatment on cells present in the epidermis.

Specific examples of the physical or chemical treatment include one or more selected from the group consisting of chemical peeling, alkali cleansing treatment, depilatory waxing, dermabrasion, microdermabrasion, microneedling, radio-frequency microneedling, laser treatment, IPL treatment, cryotherapy (cooling treatment), tattoo treatment, tattoo removal, acne treatment, LED treatment, enzyme mask treatment and plasma fibroblast.

Chemical peeling is a chemical treatment of the epidermis which treats the epidermis with a composition containing alpha-hydroxy acids such as glycolic acid and lactic acid, salicylic acid, trichloroacetic acid, Baker-Gordon's solution, phenol, etc. Chemical peeling is said to eliminate or obscure acne, spots, sunburn spots, wrinkles, and the like. After a chemical peeling composition is applied, inflammation, redness, etc., occur in the epidermis.

Alkaline cleansing treatment is a chemical treatment which changes the pH of the epidermis rapidly from about 5.6 to about 12. This treatment is said to eliminate or obscure acne, spots, wrinkles, and the like. This alkaline treatment can also cause inflammation, redness, etc., in the epidermis.

Depilatory waxing is one of the depilation means, and is a treatment for removing hair by applying wax to a site to be depilated, and then peeling off the solidified wax. When the solidified wax is peeled off, physical irritation can cause inflammation, redness, etc., in the epidermis.

Dermabrasion is an exfoliating technique and uses a rotating instrument to remove the outer layers of skin. Numb with anesthesia will be used before removing the damaged outermost layers of the skin during the procedure. Dermabrasion can also help treat acne scars, age spots, wrinkles, precancerous skin patches, rhinophyma, scars from surgery or injury, sun damage, tattoos, uneven skin tone, etc.

Meanwhile, microdermabrasion is a minimally invasive procedure for renewing overall skin tone and texture. A special applicator with an abrasive surface will be used to gently sand away the thick outer layer of the skin to rejuvenate it. It can improve the appearance of fine lines, wrinkles, hyperpigmentation, age spots, brown spots, enlarged pores, blackheads, acne, acne scars, stretch marks, dull-looking skin complexion, uneven skin tone and texture, melasma, sun damage, and other skin-related concerns and conditions.

Microneedling and radio-frequency microneedling are methods of making wounds on the skin with tiny needles to promote skin regeneration, and are used for acne scarring improvement and skin rejuvenation. These systems can also cause inflammation, redness, pain, etc.

Laser treatment is a method of treating skin with a laser characterized with special range of wavelength such as Q-switched laser with a nanosecond pulse width, and is used for improving pigmented lesions, such as spots, and rejuvenating the skin. This treatment can cause inflammation, redness, swelling, etc.

IPL (intense pulsed light) treatment is a light treatment which irradiates the skin with special light called IPL, and is used for spots, freckles, wrinkles, acne, and the like. This treatment can also cause inflammation etc. of the skin.

Cryotherapy is also called cooling treatment. In cryotherapy, using liquid nitrogen ranging from -120°C to -196°C is applied for a certain period of time for spots, freckles, acne, and the like. This treatment can cause inflammation, redness, pain, etc., in the skin.

Tattoo treatment is a means for drawing characters and pictures by inserting pigments into the skin and tattoo removal is a means for removing them. These treatments can also cause inflammation, redness, swelling, etc.

Acne treatment can be, for example, performed according to the following steps.

### Facial Step 1: Deep cleansing

This step starts with removing the makeup and performing a thorough cleansing. After the first cleansing, cleanse the skin again, and make sure that stains, oil and cosmetic traces have been completely removed.

### Facial Step 2: Steam treatment

A special steamer maker flows warm steam over the face for a few minutes. What the steam actually does is to soften the pores along with the horn plug made of the sebum in the pores, and to make the face easier to cleanse.

### Facial Step 3: Exfoliating procedure

The exfoliating procedure serves to removing dead skin cells and debris that can potentially clog the pores. There are many options for exfoliating, from simple scrubs to fine dermabrasion or superficial chemical exfoliation. Peeling with salicylic acid is commonly used during facial acne treatment.

### Facial Step 4: Extracting spots

Aestheticians manually cleanse to remove acnes in the pores. This can be done by applying a friendly pressure with fingers or by using a small device called an acne extractor.

### Facial Step 5: Wearing the Mask

After the extraction is completed, wear the mask. Sulfur serves to removing acne, and so sulfur masks are often used in the acne treatment. For a super-oily skin type, an oil-absorbing clay mask may be used instead. A smoothing mask is the best choice if the skin is a little reddish from the inflammation acne or the extract liquor.

### Facial Step 6: Cosmetic water or astringent

Once the mask is removed, apply cosmetic water or an astringent to the entire skin.

### Facial Step 7: Moisturizer and sunscreen

Apply a mild, non-acne moisturizer to the face, the neck, and the chest, overall. Apply sunscreen after the treated area is applied with thin fibers.

Among the above acne treatments, when a physical cleansing means such as pore washing is used, inflammation etc. can occur.

The LED treatment employs a blue light having a wavelength of 415-420 nm and/or a red light having a wavelength of 610-850 nm. The blue light is said to be mainly effective for acne. The red light is known to promote collagen generation, and is said to be effective for anti-aging. These LED treatments can cause inflammation etc. on the skin.

The enzyme mask treatment is a skin treatment with a facial mask containing protease and the like. The enzyme mask treatment can remove proteins with dead epidermis and toxins. This enzyme mask treatment can cause inflammation etc.

The step B) is carried out after the above step A) above, and is a step forming a film containing a fiber deposit on the skin which has been subjected to the step A) .

The film formed in the step B) is a film containing a fiber deposit. The fibers contained in the film preferably have an average fiber diameter of 0.01 µm or more and 7 µm or less. The average fiber diameter is more preferably 0.05 µm or more, and even more preferably 0.1 µm or more; and more preferably 5 µm or less, and even more preferably 3 µm or less. Use of the film formed in the step B) characterized with a deposit of such thin fibers improves skin conditions such as inflammation, redness, and swelling, which may be caused by the step A), promotes their healing, improves the adhesiveness of the film to the skin, and improves the ability to follow the movement of the skin.

Here, the average fiber diameter is the average thickness of fibers, and is equivalent to circle diameter. The thickness of fibers can be measured, for example, by observing the fibers using scanning electron microscope (SEM) observation at a magnification of 10,000 times, after randomly selecting 10 fibers, except for defects (lumps of fibers, intersections of fibers, and droplets), from the two-dimensional images, drawing a line orthogonal to the longitudinal direction of each fiber, and directly reading the fiber diameter.

The fiber length is not particularly limited, but is preferably at least 10 times, more preferably at least 20 times, and even more preferably at least 50 times longer than that of the average fiber diameter. The upper limit of the fiber length is not particularly limited. Fibers with a fiber length at least 100 times longer than the average fiber diameter are defined as successive fibers, and any successive fibers may be used.

The basis weight of the film containing a fiber deposit is preferably 0.1 g/m² or more, and more preferably 1 g/m² or more. Moreover, the basis weight of the film is preferably 30 g/m² or less, and more preferably 20 g/m² or less. For example, the basis weight of the film is preferably 0.1 g/m² or more and 30 g/m² or less, and more preferably 1 g/m² or more and 20 g/m² or less. By setting the basis weight of the film as described above, the adhesiveness of the film can be improved.

Step B) is not particularly limited as long as it is a means capable of forming the film containing a fiber deposit on the skin. Specific examples thereof include the following means (1) to (3):
(1) A step of electrostatically spraying a composition X comprising the following components (a) and (b) to the skin:
   (a) one or more volatile substances selected from the group consisting of water, alcohols, and ketones; and
   (b) a polymer having an ability to form a film containing a fiber deposit.
(2) A step of attaching a film containing a fiber deposit having an average fiber diameter ranging from 0.01 to 7 µm by the use of electrospinning.
(3) A step of applying a composition Z comprising the following components (c) and (d):
   (c) one or more selected from the group consisting of an oil agent and a polyol which are in a liquid state at 20°C; and
   (d) a fiber having an average fiber diameter ranging from 0.01 to 7 µm and an aspect ratio (average fiber length/average fiber diameter) ranging from 10 to 1,000.

First, the means (1) is described.

The method for forming a film used in the means (1) is an electrostatic spray method. The electrostatic spray method is a method in which a positive or negative high voltage is applied to a composition to charge the composition, and the charged composition is sprayed toward an object. The sprayed composition spreads onto the space while repeating miniaturization by Coulomb repulsion. During that process or after attachment to the object, the solvent, which is a volatile substance, is dried to thereby form a film containing a fiber deposit on the surface of the object. This means (1) can be carried out using the methods and devices disclosed, for example, in WO 2018/194143, WO 2018/194140, and WO 2019/103974.

The component (a) contained in the composition X used in the means (1) is one or more volatile substances selected from the group consisting of water, an alcohol, and a ketone.

The volatile substance as the component (a) is a substance which is volatile in the liquid state. The component (a) is mixed in the composition X for the purpose that the component (a) is discharged from the tip of a nozzle toward the skin after the composition X placed in the electric field is sufficiently charged; as the component (a) evaporates, the charge density of the composition X becomes excessive; the component (a) further evaporates while being further miniaturized due to Coulomb repulsion; and finally a dry film containing a fiber deposit is formed on the skin. For this purpose, the vapor pressure of the volatile substance at 20°C is preferably 0.01 kPa or more and 106.66 kPa or less, more preferably 0.13 kPa or more and 66.66 kPa or less, further more preferably 0.67 kPa or more and 40.00 kPa or less, and even more preferably 1. 33 kPa or more and 40.00 kPa or less.

Among the volatile substances as the component (a), examples of preferably used alcohols include monovalent chain aliphatic alcohols, monovalent cyclic aliphatic alcohols, and monovalent aromatic alcohols. Examples of the monovalent chain aliphatic alcohols include C₁-C₆ alcohols, examples of the monovalent cyclic aliphatic alcohols include C₄-C₆ cyclic alcohols, and examples of the monovalent aromatic alcohols include benzyl alcohol, and phenylethyl alcohol. Specific examples thereof include ethanol, isopropyl alcohol, butyl alcohol, phenylethyl alcohol, n-propanol, and n-pentanol. One or more of these alcohols can be used.

Among the volatile substances as the component (a), examples of ketones include di-C₁-C₄ alkyl ketones, such as acetone, methyl ethyl ketone, and methyl isobutyl ketone. These ketones can be used singly or in combination of two or more.

The volatile substance as the component (a) is more preferably one or more selected from the group consisting of ethanol, isopropyl alcohol, butyl alcohol, and water; more preferably one or more selected from the group consisting of ethanol and butyl alcohol; and even more preferably a volatile substance containing at least ethanol.

The volatile substance as the component (a) more preferably contains water, from the viewpoint of imparting charge. The water content based on the total amount of the volatile substances is preferably 0.01% by mass or more, and more preferably 0.04% by mass or more, from the viewpoint of imparting charge; and is preferably 10% by mass or less, and more preferably 5% by mass or less, from the viewpoint of fiber-forming properties.

From the viewpoint of fiber-forming properties, the content of the component (a) in the composition X is preferably 30% by mass or more, more preferably 55% by mass or more, and even more preferably 60% by mass or more. Moreover, from the viewpoint of fiber-forming properties, the content of the component (a) in the composition X is preferably 98% by mass or less, more preferably 96% by mass or less, and even more preferably 94% by mass or less. When an oil, a polyol, or the like is contained, the content of the component (a) is preferably 90% by mass or less, and more preferably 85% by mass or less. The content of the component (a) in the composition X is preferably 30% by mass or more and 98% by mass or less, more preferably 55% by mass or more and 96% by mass or less, and even more preferably 60% by mass or more and 94% by mass or less. Moreover, when the composition X contains a polyol or an oil, the content of the component (a) in the composition X is preferably 30% by mass or more and 90% by mass or less, more preferably 55% by mass or more and 90% by mass or less, and even more preferably 60% by mass or more and 90% by mass or less. It is preferable that the composition X comprises the component (a) at this ratio, from the viewpoint of the volatility of the composition X when an electrostatic spray method is carried out.

Further, the amount of ethanol is preferably 50% by mass or more, more preferably 65% by mass or more, and even more preferably 80% by mass or more, based on the total amount of the volatile substances as the component (a). Moreover, the amount of ethanol is preferably 100% by mass or less. The amount of ethanol is preferably 50% by mass or more and 100% by mass or less, more preferably 65% by mass or more and 100% by mass or less, and even more preferably 80% by mass or more and 100% by mass or less, based on the total amount of the volatile substances as the component (a).

The polymer having an ability to form a film containing a fiber deposit as the component (b) is generally a substance which can be dissolved in the volatile substance as the component (a). The term "to dissolve" as mentioned herein means that the substance is in a dispersed state at 20°C, and that the dispersed state is visually uniform, and preferably visually transparent or translucent.

As the polymer having an ability to form a film containing a fiber deposit (b), suitable polymers are used depending on the properties of the volatile substance as the component (a). Specifically, such polymers are roughly divided into water-soluble fiber-forming polymers and water-insoluble fiber-forming polymers. In the present specification, the "water-soluble polymers" refer to those having the following property: 1 g of polymer is weighed in an environment of 1 atm and 23°C, then immersed in 10 g of ion-exchanged water, and after 24 hours, 0.5 g or more of the immersed polymer is dissolved in water. Meanwhile, in the present specification, the "water-insoluble polymers" refer to those having the following property: 1 g of polymer is weighed in an environment of 1 atm and 23°C, then immersed in 10 g of ion-exchanged water, and after 24 hours, 0.5 g or more of the immersed polymer is not dissolved in water.

Examples of the water-soluble fiber-forming polymers include mucopolysaccharides, such as pullulan, hyaluronic acid, chondroitin sulfate, poly-γ-glutamic acid, modified cornstarch, β-glucan, gluco-oligosaccharide, heparin, and keratosulfate; natural polymers, such as cellulose, pectin, xylan, lignin, glucomannan, galacturonic acid, psyllium seed gum, tamarind seed gum, gum arabic, tragacanth gum, soybean water-soluble polysaccharide, alginic acid, carrageenan, laminaran, agar (agarose), fucoidan, methylcellulose, hydroxypropyl cellulose, and hydroxypropyl methylcellulose; and synthetic polymers, such as partially-saponified polyvinyl alcohol (when not used in combination with a crosslinking agent), low-saponified polyvinyl alcohol, water-soluble nylon, polyvinyl pyrrolidone (PVP), polyethylene oxide, and sodium polyacrylate. These water-soluble polymers can be used singly or in combination of two or more. Among these water-soluble polymers, from the viewpoint of easy production of the film, it is preferable to use one or more selected from the group consisting of pullulan and synthetic polymers, such as partially-saponified polyvinyl alcohol, low-saponified polyvinyl alcohol, water-soluble nylon, polyvinyl pyrrolidone, and polyethylene oxide. When polyethylene oxide is used as the water-soluble polymer, the number average molecular weight thereof is preferably 50,000 or more and 3,000,000 or less, and more preferably 100,000 or more and 2,500,000 or less.

Meanwhile, examples of the water-insoluble fiber-forming polymers include completely-saponified polyvinyl alcohol insolubilizable after the formation of the film; partially-saponified polyvinyl alcohol crosslinkable used in combination with a crosslinking agent after the formation of the film; oxazoline-modified silicones, such as poly(N-propanoylethyleneimine) graft-dimethylsiloxane/y-aminopropylmethylsiloxane copolymer; polyvinylacetal diethylaminoacetate; Zein (main component of corn protein); polyester resins, such as polyester and polylactic acid (PLA); acrylic resins, such as polyacrylonitrile resin and polymethacrylic acid resin; polystyrene resins, polyvinyl butyral resins, polyethylene terephthalate resins, polybutylene terephthalate resins, polyurethane resins, polyamide resins, polyimide resins, and polyamideimide resins. These water-insoluble polymers can be used singly or in combination of two or more. Among these water-insoluble polymers, it is preferable to use one or more selected from the group consisting of completely-saponified polyvinyl alcohol insolubilizable after the formation of the film, partially-saponified polyvinyl alcohol crosslinkable used in combination with a crosslinking agent after the formation of the film, polyvinyl butyral resins, polyurethane resins, oxazoline-modified silicones, such as poly(N-propanoylethyleneimine) graft-dimethylsiloxane/y-aminopropylmethylsiloxane copolymer, polyvinylacetal diethylaminoacetate, and Zein; and it is more preferable to use one or more selected from the group consisting of polybutyral resins and polyurethane resins.

The content of the component (b) in the composition X is preferably 2% by mass or more, more preferably 4% by mass or more, and even more preferably 6% by mass or more. Moreover, the content of the component (b) in the composition X is preferably 50% by mass or less, more preferably 45% by mass or less, and even more preferably 40% by mass or less. The content of the component (b) in the composition X is preferably 2% by mass or more and 50% by mass or less, more preferably 4% by mass or more and 45% by mass or less, and even more preferably 6% by mass or more and 40% by mass or less. It is preferable to mix the component (b) into the composition X at this ratio, from the viewpoint of composing a fiber deposit, masking the surface of the skin, alleviating various skin conditions of the skin which has been subjected to the step A), and promoting healing them.

The content ratio of the component (a) and the component (b), ((a)/(b)), in the composition X is preferably 0.5 or more and 40 or less, more preferably 1 or more and 30 or less, and even more preferably 1.3 or more and 25 or less, from the viewpoint that the component (a) can be sufficiently volatilized when an electrostatic spray method is carried out.

The content ratio of ethanol and the component (b), (ethanol/(b)), in the composition X is preferably 0.5 or more and 40 or less, more preferably 1 or more and 30 or less, and even more preferably 1.3 or more and 25 or less, from the viewpoint that ethanol can be sufficiently volatilized when an electrostatic spray method is carried out.

The composition X may contain glycol. Examples of glycols include ethylene glycol, propylene glycol, butylene glycol, diethylene glycol, dipropylene glycol, and polypropylene glycol. From the viewpoint that the component (a) can be sufficiently volatilized when an electrostatic spray method is carried out, the content of glycol in the composition X is preferably 10% by mass or less, and more preferably 8% by mass or less.

From the viewpoint of fiber-forming properties and conductivity, the water content is preferably less than 50% by mass, more preferably 45% by mass or less, further more preferably 10% by mass or less, and even more preferably 5% by mass or less; and preferably 0.2% by mass or more, and more preferably 0.4% by mass or more, based on the total amount of the volatile substances as the component (a).

The composition X may contain a powder. Examples of powders include color pigments, extender pigments, pearl pigments, and organic powder. In terms of imparting a smooth feel to the skin surface, the content of the powder in the composition X is preferably 5% by mass or less, more preferably 3% by mass or less, and even more preferably 1% by mass or less; the powder is preferably substantially not contained.

The composition X may contain only the components (a) and (b) described above, or may contain other components in addition to the components (a) and (b). Examples of other components include oil agents such as di(phytosteryl/octyldodecyl) lauroyl glutamate, polyols which are liquid at 20°C other than those mentioned above, such as glycerol, surfactants, UV protective agents, flavoring agents, repellents, antioxidants, stabilizers, antiseptics, antiperspirant, and various vitamins. Each of these agents is not limited to the intended use of each agent, and can be used for other applications depending on the purpose; for example, an antiperspirant can be used as a flavoring agent. Alternatively, as combined use with other applications, for example, they can be used as those having an effect as an antiperspirant and an effect as a flavoring agent. When the composition X contains other components, the content ratio of the other components is preferably 0.1% by mass or more and 30% by mass or less, and more preferably 0.5% by mass or more and 20% by mass or less.

In the means (1), after the step A), the composition X is directly electrostatically sprayed to the skin to form a film containing a fiber deposit on the skin surface.

When an electrostatic spray method is carried out, the composition X used has a viscosity at 25°C of preferably 1 mPa·s or more, more preferably 10 mPa·s or more, and even more preferably 50 mPa·s or more. Further, the viscosity at 25°C is preferably 5,000 mPa·s or less, more preferably 2,000 mPa·s or less, and even more preferably 1,500 mPa·s or less. The viscosity of the composition X at 25°C is preferably 1 mPa·s or more and 5,000 mPa·s or less, more preferably 10 mPa·s or more and 2,000 mPa·s or less, and even more preferably 50 mPa·s or more and 1,500 mPa·s or less. Use of the composition X having a viscosity in these ranges facilitate to form a porous film containing a fiber deposit by an electrostatic spray method. The formation of a porous film containing a fiber deposit is advantageous from the viewpoint of masking the surface of the skin, alleviating various skin conditions of the skin which has been subjected to the step A), and promoting healing them. The viscosity of the composition X is measured at 25°C using an E-type viscometer. Examples of the E-type viscometer suitable for use include an E-type viscometer manufactured by Tokyo Keiki Inc. The rotor suitable for that case is rotor No. 43.

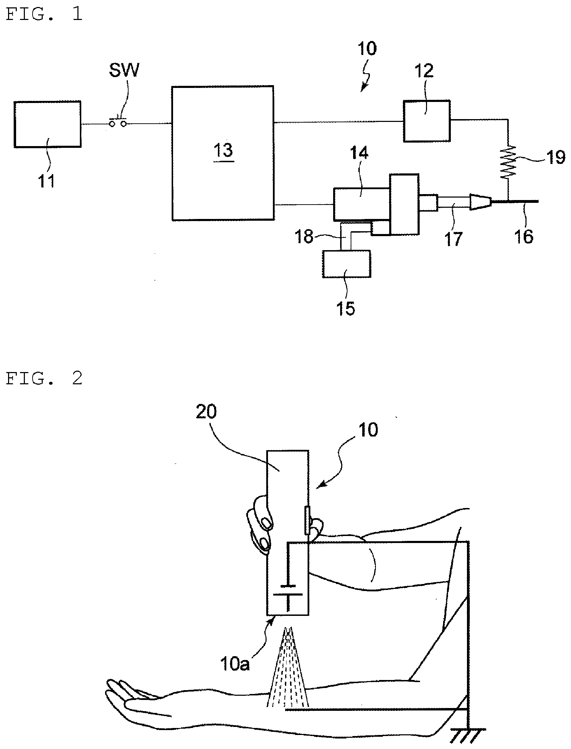

The composition X is directly sprayed to the human skin by an electrostatic spray method. The electrostatic spray method comprises a step of forming a film by electrostatically spraying a spray composition to the skin using an electrostatic spray device in an electrostatic spraying process. The electrostatic spray device is equipped with a container for containing the spray composition, a nozzle for discharging the spray composition, a supply unit for supplying the spray composition contained in the container to the nozzle, and a power supply for applying a voltage to the nozzle. FIG. 1 shows a schematic diagram showing the structure of an electrostatic spray device suitably used in the present invention. The electrostatic spray device 10 shown in FIG. 1 is equipped with a low-voltage power supply 11. The low-voltage power supply 11 is capable of generating a voltage of several V to ten-odd V. For the purpose of enhancing the portability of the electrostatic spray device 10, the low-voltage power supply 11 preferably comprises one or more batteries. Further, the use of a battery(s) as the low-voltage power supply 11 is advantageous in that they can be easily replaced if necessary. Instead of a battery(s), an AC adapter or the like can also be used as the low-voltage power supply 11. FIG. 2 shows a situation of electrostatic spraying on one's own skin.

Next, the means (2) is described.

The means (2) is a step of attaching a film containing a fiber deposit having an average fiber diameter ranging from 0.01 to 7 µm obtained by electrospinning or melt blow.

The film containing a fiber deposit having an average fiber diameter ranging from 0.01 to 7 µm used in the means (2) is preferably one obtained by electrospinning or melt blow of the composition X comprising the components (a) and (b) on a substrate. The electrospinning means can also be carried out in the same manner as in the electrostatic spray means. However, the voltage etc. during electrospinning may be high, because electrospinning is not performed on the skin. The substrate used is a metal, a resin, or the like. In addition, the melt blow means can melt a resin at a temperature equal to or higher than the melting point, and discharge the molten resin while also discharging hot air around the discharge port to form a film containing a fiber deposit having the above-mentioned average fiber diameter.

The film composed of a fiber deposit having an average fiber diameter ranging from 0.01 to 7 µm obtained by electrospinning or melt blow may be attached to the skin which has been subjected to the step A).

When the means (1) or (2) is employed, it is preferable to perform a step C) of applying a composition Y comprising one or more selected from the group consisting of an oil agent and a polyol before or after the step B), from the viewpoint of imparting more excellent transparency to the film formed in the step B) and improving durability (abrasion resistance, stretchability, etc.). The composition Y may further contain an adhesive polymer, from the viewpoint of improving the durability of the film formed in the step B).

Examples of the oil agent contained in the composition Y include one or more selected from the group consisting of liquid oils (oils in a liquid state at 20°C) and solid oils (oils in a solid state at 20°C) .

The liquid oils in the present invention also include oils in a liquid state and oils in a flowable semi-solid state at 20°C. Examples of the liquid oils include hydrocarbon oils, ester oils, higher alcohols, silicone oils, and fatty acids. Among these, in terms of smoothness during application, and the abrasion resistance and stretchability of the film, hydrocarbon oils, ester oils, and silicone oils are preferable. Further, one or more of these liquid oils can be used in combination.

Examples of the liquid hydrocarbon oils include liquid paraffin, squalane, squalene, n-octane, n-heptane, cyclohexane, light isoparaffin, liquid isoparaffin, hydrogenated polyisobutene, polybutene, and polyisobutene. From the viewpoint of usability, liquid paraffin, light isoparaffin, liquid isoparaffin, squalane, squalene, n-octane, n-heptane, and cyclohexane are preferable; and liquid paraffin and squalane are more preferable. Further, from the viewpoint of the abrasion resistance and stretchability of the film, the viscosity of hydrocarbon oils at 30°C is preferably 1 mPa·s or more, and more preferably 3 mPa·s or more. Moreover, from the viewpoint of the abrasion resistance and stretchability of the film, the total content of isododecane, isohexadecane, and hydrogenated polyisobutene in the liquid agent is preferably 10% by mass or less, more preferably 5% by mass or less, even more preferably 1% by mass or less, and further more preferably 0.5% by mass or less, and they may not be contained.

Similarly, from the viewpoint of the abrasion resistance and stretchability of the film, the viscosity of ester oils and silicone oils at 30°C is preferably 1 mPa·s or more, and more preferably 3 mPa·s or more.

The viscosity herein is measured at 30°C using a BM-type viscometer (manufactured by Tokimec Inc., measurement conditions: rotor No.1, 60 rpm, 1 minute). From the similar viewpoint, the total content of ether oils (e.g., cetyl-1,3-dimethylbutyl ether, dicapryl ether, dilauryl ether, and diisostearyl ether) in the liquid agent is preferably 10% by mass or less, more preferably 5% by mass or less, and even more preferably 1% by mass or less.

Examples of the ester oils include esters containing linear or branched fatty acids and linear or branched alcohols or polyhydric alcohols. Specific examples thereof include isopropyl myristate, cetyl isooctanoate, isocetyl octanoate, octyldodecyl myristate, isopropyl palmitate, butyl stearate, hexyl laurate, decyl oleate, octyldodecyl oleate, hexyldecyl dimethyloctanoate, cetyl lactate, myristyl lactate, lanolin acetate, isocetyl stearate, isocetyl isostearate, ethylhexyl isononanoate, isononyl isononanoate, isotridecyl isononanoate, isostearyl isostearate, cholesteryl 12-hydroxystearate, ethylene glycol di-2-ethylhexanoate, dipentaerythritol fatty acid ester, N-alkyl glycol monoisostearate, propylene glycol dicaprylate, propylene glycol diisostearate, neopentyl glycol dicaprate, diisostearyl malate, glycerol di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, trimethylolpropane triisostearate, cetyl-2-ethylhexanoate, 2-ethylhexyl palmitate, diethylhexyl naphthalene dicarboxylate, (C₁₂-C₁₅) alkyl benzoate, cetearyl isononanoate, caprylic/capric triglyceride, butylene glycol dicaprylate/caprate, propylene glycol dicaprylate/dicaprate, glyceryl triisostearate, glyceryl tri-2-heptylundecanoate, glyceryl tricocoate, methyl castorate, oleyl oleate, 2-heptylundecyl palmitate, diisobutyl adipate, 2-octyldodecyl N-lauroyl-L-glutamate, di-2-heptylundecyl adipate, ethyl laurate, di-2-etylhexyl sebacate, 2-hexyldecyl myristate, 2-hexyldecyl palmitate, 2-hexyldecyl adipate, diisopropyl sebacate, di-2-ethylhexyl succinate, triethyl citrate, 2-ethylhexyl paramethoxycinnamate, and tripropylene glycol dipivalate.

Among these, from the viewpoint of closely attaching the film to the skin, and in terms of an excellent feeling during application to the skin, at least one is preferably selected from the group consisting of octyldodecyl myristate, myristyl myristate, isocetyl stearate, isononyl isononanoate, isocetyl isostearate, cetearyl isononanoate, diisobutyl adipate, di-2-etylhexyl sebacate, isopropyl myristate, isopropyl palmitate, diisostearyl malate, neopentylglycol dicaprate, (C₁₂-C₁₅)alkyl benzoate, and caprylic/capric triglyceride; more preferably at least one selected from the group consisting of isopropyl myristate, isopropyl palmitate, diisostearyl malate, neopentylglycol dicaprate, (C₁₂-C₁₅)alkyl benzoate, and caprylic/capric triglyceride; and even more preferably at least one is selected from the group consisting of neopentylglycol dicaprate and caprylic/capric triglyceride.

Moreover, as the ester oils, vegetable oils and animal oils containing the above ester oils can also be used. Examples thereof include olive oil, jojoba oil, macadamia nut oil, meadowfoam oil, castor oil, safflower oil, sunflower oil, avocado oil, canola oil, apricot oil, rice embryo oil, and rice bran oil.

Examples of the higher alcohols include liquid higher alcohols having 12 to 20 carbon atoms, and higher alcohols containing a branched chain fatty acid as a constituent are preferable. Specific examples thereof include isostearyl alcohol, and oleyl alcohol.

The liquid silicone oil is, for example, linear silicone, cyclic silicone, or modified silicone. Examples thereof include dimethylpolysiloxane, dimethylcyclopolysiloxane, methylphenylpolysiloxane, methylhydrogenpolysiloxane, phenyl-modified silicone, and higher alcohol-modified organopolysiloxane.

Further, an oil agent which is solid at 20°C (solid oil) can also be used. The oil agent solid at 20°C is preferably one which exhibits solid properties at 20°C and has a melting point of 40°C or higher. Examples of the oil agent solid at 20°C include hydrocarbon wax, ester wax, parahydroxybenzoic acid esters, higher alcohols, linear fatty acid esters having 14 or more carbon atoms, triglycerides containing three linear fatty acids having 12 or more carbon atoms as constituents, and silicone wax. One or more of these oil agents can be contained. Such waxes are not limited as long as they are used in ordinary cosmetics. Examples thereof include mineral-based waxes, such as ozokerite and ceresin; petroleum-based waxes, such as paraffin, microcrystalline wax, and petrolatum; synthetic hydrocarbon waxes, such as Fischer-Tropsch wax and polyethylene wax; plant-based waxes, such as carnauba wax, candelilla wax, rice wax, Japan wax, sunflower wax, and hydrogenated jojoba oil; animal-based waxes, such as beeswax and whale wax; synthetic waxes, such as silicone wax, fluorine-based wax, and synthetic beeswax; fatty acids, higher alcohols, and the derivatives thereof. Moreover, examples of parahydroxybenzoic acid esters include methyl parahydroxybenzoate, ethyl paraaminobenzoate, isobutyl parahydroxybenzoate, isopropyl parahydroxybenzoate, ethyl parahydroxybenzoate, butyl parahydroxybenzoate, propyl parahydroxybenzoate, and benzyl parahydroxybenzoate. Examples of triglycerides containing three linear fatty acids having 12 or more carbon atoms as constituents include glyceryl trilaurate, glyceryl trimyristate, glyceryl tripalmitate, and glyceryl tribehenate. Examples of fatty acid ester oils containing linear fatty acids having 14 or more carbon atoms as constituents include myristyl myristate.

In terms of the abrasion resistance, stretchability, and usability of the film, the content of the oil agent in the composition Y is preferably 1% by mass or more and 20% by mass or less, more preferably 2% by mass or more and 18% by mass or less, and even more preferably 3% by mass or more and 16% by mass or less.

Examples of the polyol used in the composition Y include alkylene glycols, such as ethylene glycol, propylene glycol, 1,3-propanediol, and 1,3-butanediol; polyalkylene glycols, such as diethylene glycol, dipropylene glycol, polyethylene glycol with a molecular weight of 1,000 or less, and polypropylene glycol; and glycerols, such as glycerol, diglycerol, and triglycerol. Among these, from the viewpoint of the abrasion resistance and stretchability of the film, ethylene glycol, propylene glycol, 1,3-butanediol, dipropylene glycol, polyethylene glycol with a molecular weight of 1,000 or less, glycerol, and diglycerol are preferable; propylene glycol, 1,3-butanediol, and glycerol are more preferable; and glycerols are even more preferable.

From the viewpoint of the abrasion resistance and stretchability of the film, the content of the polyol in the composition Y is preferably 1% by mass or more and 40% by mass or less, more preferably 1% by mass or more and 30% by mass or less, further more preferably 3% by mass or more and 25% by mass or less, even more preferably 5% by mass or more and 20% by mass or less, and further even more preferably 10% by mass or more and 20% by mass or less.

The adhesive polymer used in the composition Y contributes to the improvement of the abrasion resistance and stretchability of the film formed on the skin by electrostatic spraying. As the adhesive polymer, those generally used as adhesives or pressure-sensitive adhesives can be used. Examples thereof include rubber-based adhesive polymers, silicone-based adhesive polymers, acrylic-based adhesive polymers, and urethane-based adhesive polymers. One or more of these polymers can be used. Moreover, as the adhesive polymer, at least one selected from the group consisting of nonionic polymers, anionic polymers, cationic polymers, and amphoteric polymers can be used. The adhesive polymer is preferably a polymer other than the polymer as the component (b).

The adhesive polymer to be selected has good adhesiveness, from the viewpoint of improving the abrasion resistance and stretchability of the film. The adhesive polymer is preferably a polymer having a maximum tensile shear load of 1 N or more, more preferably 3 N or more, and even more preferably 5 N or more, measured with reference to JIS K6850. From the viewpoint of ensuring adhesiveness, a polymer having a maximum tensile shear load of 8 N or more is further more preferable. Moreover, the maximum tensile shear load is preferably 200 N or less, more preferably 150 N or less, and even more preferably 100 N or less. Specifically, it is preferable to use one or more of rubber-based adhesive polymers, silicone-based adhesive polymers, acrylic-based adhesive polymers, and urethane-based adhesive polymers; and it is preferable to use at least one selected from the group consisting of nonionic polymers, anionic polymers, cationic polymers, and amphoteric polymers.

The adhesiveness (maximum tensile shear load) of the polymer can be measured as follows. Twenty mg of a polymer solution (10% ethanol solution or saturated solution) is applied to one polycarbonate substrate (manufactured by Standard Test Piece, Carboglass Polish Clear, 10 cm x 2.5 cm x 2.0 mm) at the edge in the area of 1.25 cm x 2.5 cm. This substrate is bonded together with another polycarbonate substrate, and dried for 12 hours or more. Tensilon UTC-100W manufactured by Orientec Co., Ltd. is used to pull both ends of the polycarbonate substrates at a tensile speed of 5 mm/min, and the maximum tensile shear load is measured.

When the composition Y contains an adhesive polymer, the content thereof is preferably 5% by mass or more and 20 % by mass or less, and more preferably 6% by mass or more, from the viewpoint of the abrasion resistance and stretchability of the film. Moreover, the content of the adhesive polymer is more preferably 15% by mass or less, even more preferably 12% by mass or less, and further more preferably 10% by mass or less. Specifically, the content of the adhesive polymer is preferably 5% by mass or more and 15% by mass or less, more preferably 5% by mass or more and 12% by mass or less, and even more preferably 5% by mass or more and 10% by mass or less.

The step of applying the composition Y to the skin (step C)) may be performed before or after the step B). Moreover, examples of the means for applying the composition Y to the skin include a means for applying the composition Y to the skin with fingers or the like, a means for applying the composition Y to the skin using an applicator, and the like. The composition X and the composition Y have different formations. The composition X to be subjected to electrospinning and the composition Y to be applied to the skin before or after that are separately formulated with different formulations. Even if the composition Y is electrostatically sprayed to form a film, a deposit containing the same fibers as the composition X is not formed.

Next, the means (3) is described.

The means (3) is a step of applying a composition Z comprising the following components (c) and (d):
(c) one or more selected from the group consisting of oil agents and polyols which are liquid at 20°C; and
(d) a fiber having an average fiber diameter ranging from 0.01 to 7 µm and an aspect ratio (average fiber length/average fiber diameter) ranging from 10 to 1,000.

Examples of the oil agent and polyol as the component (c) used in the composition Z include the oil agents and polyols used in the composition Y described above.

That is, examples of the oil agent contained in the composition Z include one or more selected from the group consisting of liquid oils (oils in a liquid state at 20°C) and solid oils (oils in a solid state at 20°C) .

These liquid oils also include oils in a liquid state and oils in a flowable semi-solid state at 20°C. Examples of the liquid oils include a hydrocarbon oil, an ester oil, a higher alcohol, a silicone oil, and a fatty acid. Among these, in terms of smoothness during application, and the abrasion resistance and stretchability of the film, a hydrocarbon oil, an ester oil, and a silicone oil are preferable. Further, one or more of these liquid oils can be used in combination. Specific examples of the hydrocarbon oil, the ester oil, the higher alcohol, the silicone oil, and the fatty acid also include those mentioned above as examples used in the composition Y.

In terms of the abrasion resistance, stretchability, and usability of the film, the content of the oil agent in the composition Z is preferably 1% by mass or more and 20% by mass or less, more preferably 2% by mass or more and 18% by mass or less, and even more preferably 3% by mass or more and 16% by mass or less.

Examples of the polyol used in the composition Z include alkylene glycols such as ethylene glycol, propylene glycol, 1,3-propanediol, and 1,3-butanediol; polyalkylene glycols such as diethylene glycol, dipropylene glycol, polyethylene glycol with a molecular weight of 1,000 or less, and polypropylene glycol; and glycerols such as glycerol, diglycerol, and triglycerol. Among these, from the viewpoint of the abrasion resistance and stretchability of the film, ethylene glycol, propylene glycol, 1,3-butanediol, dipropylene glycol, polyethylene glycol with a molecular weight of 1,000 or less, glycerol, and diglycerol are preferable; propylene glycol, 1,3-butanediol, and glycerol are more preferable; and glycerols are even more preferable.

From the viewpoint of the abrasion resistance and stretchability of the film, the content of the polyol in the composition Z is preferably 1% by mass or more and 40% by mass or less, more preferably 1% by mass or more and 30% by mass or less, even more preferably 3% by mass or more and 25% by mass or less, further more preferably 5% by mass or more and 20% by mass or less, and even further more preferably 10% by mass or more and 20% by mass or less.

It is preferable that the composition Z contains a volatile component, from the viewpoint of forming a fiber deposit on the skin when the composition Z is applied to the skin.

The volatile component used in the composition Z is preferably one or more selected from the group consisting of water, alcohols, volatile silicones, volatile hydrocarbons, and the like.

Examples of preferably used alcohols include monovalent chain aliphatic alcohols, monovalent cyclic aliphatic alcohols, and monovalent aromatic alcohols. Examples of monovalent chain aliphatic alcohols include C₁-C₆ alcohols, examples of monovalent cyclic alcohols include C₄-C₆ cyclic alcohols, and examples of monovalent aromatic alcohols include benzyl alcohol, and phenylethyl alcohol. Specific examples thereof include ethanol, isopropyl alcohol, butyl alcohol, phenylethyl alcohol, n-propanol, and n-pentanol. One or more of these alcohols can be used.

Examples of volatile silicone oils include dimethylpolysiloxane and cyclic silicone.

The content of the volatile component is preferably from 15 to 90% by mass in the composition Z, from the viewpoint of the coating properties of the composition Z and the uniformity of the film; more preferably 20% by mass or more, and even more preferably 30% by mass or more, from the viewpoint of usability when the composition Z is applied to the skin; and preferably 87% by mass or less, and more preferably 85% by mass or less, from the viewpoint of the formability of the fiber deposit after the composition Z is applied to the skin, and the durability of the film.

The fibers contained in the composition Z are (d) fibers having an average fiber diameter ranging from 0.01 to 7 µm and an aspect ratio (average fiber length/average fiber diameter) ranging from 10 to 1,000.

The component (d) forms a fiber deposit in the formed film and imparts uniformity and close adhesiveness to the film. Whether the fibers form a deposit in the film can be confirmed by a scanning electron microscope or the like. The term "deposit" refers to a state in which the fibers dispersed in the film have intersections with each other so as to have gaps, and a state in which the components contained in the composition can be retained in the gaps.

From the viewpoint of the uniformity of the formed film, the average fiber diameter of the fibers used in the composition Z is 0.01 µm or more and 7 µm or less. Moreover, from the viewpoint of the adhesiveness and fitness of the film, the average fiber diameter of the fibers is preferably 0.05 µm or more, more preferably 0.1 µm or more, further more preferably 0.2 µm or more, and even more preferably 0.3 µm or more; and preferably 5 µm or less, more preferably 4 µm or less, and even more preferably 3 µm or less. The fiber diameter can be measured, for example, by observing the fibers by scanning electron microscope observation at a magnification of from 2,000 times or 5,000 times, randomly selecting 100 fibers, except for defects (e.g., lumps of fibers and intersections of fibers), from the two-dimensional image, drawing a line orthogonal to the longitudinal direction of each fiber, and directly reading the fiber diameter. The average fiber diameter was determined by calculating the arithmetic average of these measured values.

From the viewpoint of the uniformity of the formed film, the fiber length as the average fiber length is preferably 20 µm or more and 500 µm or less, more preferably 30 µm or more, and even more preferably 40 µm or more; and more preferably 400 µm or less, further more preferably 250 µm or less, and even more preferably 200 µm or less. The average fiber length can be measured, for example, by observing the fiber length by scanning electron microscope observation at a magnification ranging from 250 times or 750 times depending on the fiber length, randomly selecting 100 fibers, except for defects (e.g., lumps of fibers and intersections of fibers), from the two-dimensional image, drawing a line in the longitudinal direction of each fiber, and directly reading the fiber length. The average fiber length was determined by calculating the arithmetic average of these measured values.

From the viewpoint of the uniformity of the formed film, and the film durability due to the formation of a uniform fiber deposit, the aspect ratio of the fibers (average fiber length/average fiber diameter) is 10 or more and 1,000 or less, preferably 15 or more, and more preferably 20 or more; and more preferably 500 or less, further more preferably 400 or less, and even more preferably 300 or less.

In the composition Z, in order for the fibers to form a deposit in the formed film, and in order to enhance the uniformity and adhesiveness of the film, the ratio of (average fiber diameter²)/average fiber content (µm²/% by mass) is preferably within the range from 0.005 to 7.

From the viewpoint of the uniformity of the fiber deposit, this value is preferably 0.02 or more, more preferably 0.03 or more, and even more preferably 0.05 or more. Further, from the viewpoint of sufficiently forming a fiber deposit in the film, this value is preferably 6 or less, more preferably 5 or less, even more preferably 4 or less, and further more preferably 3 or less. This value, i.e., the ratio of (average fiber diameter²) /average fiber content (µm²/% by mass), is an index of the cumulative length of the fibers contained in the composition Z, and means that the larger this numerical value, the shorter the cumulative length.

The fibers as the component (d) can be produced by grinding fibers obtained by treating fiber-forming polymers by various known spinning techniques. The fiber-forming polymers mentioned herein are generally thermoplastic or solvent-soluble chain polymers. Among the fiber-forming polymers, it is preferable to use a water-insoluble polymer, in terms of maintaining the shape of the fibers in the composition Z. Moreover, as the spinning method, an electrospinning method (electric field spinning method) is preferable in terms of obtaining fibers with a small fiber diameter.

In addition to the components (c) and (d) and the volatile components, the composition Z may contain surfactants, antiseptics, various powders, moisturizers other than polyols, water-soluble polymers, amino acids, dyes, and the like.

When the composition Z is applied to the skin, a film containing a highly uniform fiber deposit can be formed on the skin surface. Examples of the means for applying the composition Z to the skin include coating with fingers, coating by spraying, and coating using tools such as rollers and sponges. As a range applicable by the hand, the viscosity at 20°C is preferably ranging from 5 to 50,000 mPa·s.

As described above, when the skin is treated with the conventional step A), followed by treatment of forming a film containing a fiber deposit (step B)), conditions such as inflammation, redness, and swelling caused by the step A) are alleviated, these conditions disappear more quickly, and the quality of life (QOL) of persons who have undergone the cosmetic or dermatological treatment is significantly improved. The method of the present invention is preferably performed for cosmetic purposes.

Regarding the embodiments described above, disclosed herein are the following method, composition, and use.
<1> A method for treating skin, comprising performing the following step A) and then step B):
   A) physically or chemically treating an epidermis of the skin; and
   B) forming a film containing a fiber deposit on the skin which has been subjected to the step A).
<2> The method for treating skin according to <1>, wherein the step B) is a step of electrostatically spraying a composition X comprising the following components (a) and (b) to the skin:
   a) one or more volatile substances selected from the group consisting of water, an alcohol, and a ketone; and
   b) a polymer having an ability to form a film containing a fiber deposit.
<3> The method for treating skin according to <1>, wherein the step B) is a step of attaching a film containing a fiber deposit having an average fiber diameter ranging from 0.01 to 7 µm obtained by electrospinning or meltblowing.
<4> The method for treating skin according to any one of <1> to <3>, further comprising a step C), before or after the step B),
   C) applying a composition Y comprising one or more components selected from the group consisting of an oil agent and a polyol.
<5> The method for treating skin according to <4>, wherein the composition Y used in the step C) further comprises an adhesive polymer.
<6> The method for treating skin according to <1>, wherein the step B) is applying a composition Z comprising the following components (c) and (d):
   (c) one or more selected from the group consisting of an oil agent and a polyol which are in a liquid state at 20°C; and
   (d) a fiber having an average fiber diameter ranging from 0.01 to 7 µm and an aspect ratio (average fiber length/average fiber diameter) ranging from 10 to 1,000.
<7> The method for treating skin according to any one of <1> to <6>, wherein the step A) is a step of physically or chemically treating one or more layers selected from the group consisting of a horny layer, a granular layer, a spinous layer, and a basal layer of the skin.
<8> The method for treating skin according to any one of <1> to <7>, wherein the step A) is a skin epidermis treatment selected from the group consisting of chemical peeling, alkali cleansing treatment, depilatory waxing, dermabrasion, microdermabrasion, microneedling, radio-frequency microneedling, laser treatment, IPL treatment, cryotherapy (cooling equipment), tattoo treatment, tattoo removal, acne treatment, LED treatment, enzyme mask treatment and plasma fibroblast.
<9> The method for treating skin according to any one of <1> to <8>, wherein the polymer having an ability to form a film containing a fiber deposit (b) or a polymer constituting the fibers (d) is a water-insoluble fiber-forming polymer.
<10> Use of a composition X comprising the following components (a) and (b) for being electrostatically sprayed, after a step of physically or chemically treating an epidermis of skin, to the skin:
   a) one or more volatile substances selected from the group consisting of water, alcohols, and ketones; and
   b) a polymer having an ability to form a film containing a fiber deposit.
<11> Use of a film containing a fiber deposit having an average fiber diameter ranging from 0.01 to 7 µm obtained by electrospinning for being attached, after a step of physically or chemically treating an epidermis of skin, to the skin.
<12> Use of a composition Z comprising the following components (c) and (d) for being applied, after a step of physically or chemically treating an epidermis of skin, to the skin:
   (c) one or more selected from the group consisting of oil agents and polyols which are in a liquid state at 20°C; and
   (d) fibers having an average fiber diameter ranging from 0.01 to 7 µm and an aspect ratio (average fiber length/average fiber diameter) ranging from 10 to 1,000.
<13> A method of treating skin, comprising forming a film containing a fiber deposit on an epidermis of the skin treated physically or chemically.
<14> Use of a fiber in the manufacture of a film for alleviating stimulus on the epidermis of the skin by a physical or chemical treatment.

### Examples

Hereinafter, the present invention is described in more detail with reference to examples.

### Example 1 (chemical peeling 1)

### <Chemical peeling 1>

TCA liquid (30% by mass of trichloroacetone, 5% by mass of salicylic acid, and balanced by purified water) was used as an acid formulation, and thinly applied repeatedly from 5 to 10 times to the inside of left arm in the range of 1 cm in diameter until whitening i.e., frosting, occurred (from 1 minute to 2 minutes). Two circler areas were treated in a same manner. Subsequently, the acid formulation was wiped off from the skin with sanitary cotton. This treatment was performed by a specialist who was qualified to carry out chemical peeling.

### <Care after treatment>

Following the chemical peeling, 0.3 mL of BETA GEL (manufactured by Danne Montague-King) of DMK brand was applied.

After application of the above GEL, a fiber deposit containing nanofibers was applied to one of the treated areas under the following conditions.

Solution A for fiber formation is composed of 12% by mass of polyvinyl butyral (trade name: S-LEC B BM-1, manufactured by Sekisui Chemical Co., Ltd.), 6.8% by mass of 1,3-butylene glycol, and balancing ethanol (99.5%).

The solution A was electrostatically sprayed so as to cover the upper left inner region after chemical peeling under the following conditions.
Voltage: 14.5 kV
Flow rate: 0.08 mL/min
Distance from nozzle to skin: 14 cm
Spray time: 10 seconds
Room temperature: 20°C, Humidity: 45% RH

As described above, only the BETA GEL was applied to the other treated area, and to the area with the fiber deposit application, a care including applying the BETA GEL and the fiber deposit was applied twice a day; the care was continued until the redness and scab of the skin disappeared.

### <Results>

In the area without the fiber deposit application, the redness disappeared and the scab was removed in 20 days, whereas in the area with the fiber deposit application, the redness disappeared and the scab was removed in 8 days.

### Example 2 (chemical peeling 2)

### <Chemical peeling 2>

AHA/BHA/Retinol (14 % by mass of salicylic acid, 14 % by mass of lactic acid, 14 % by mass of retinol, and the balance: purified water) was used as an acid formulation, applied to right arm in the range of 2 cm x 2 cm, and kept for 6 hours. Three square areas were treated in a same manner. Subsequently, the acid formulation was wiped off from the skin with sanitary cotton. This treatment was performed by a specialist who was allowed to carry out chemical peeling.

### <Care after treatment>

For the two areas, the care after treatment was performed in the same manner as in Example 1, except that the amount of BETA GEL was changed to 0.5 mL, and the electrostatic spray time was changed to 30 seconds. The fiber deposit was applied onto one of the two areas. The care was performed for 1 day, and the redness disappeared 24 hours later; thus, the care was terminated.

### <Results>

Six days after the treatment, right arm above was exposed to sunlight for 6 hours. As a result, a lot of small blisters were formed on the area without any care treatment and several small blisters were formed on the area only with BETA GEL application, to which the fiber deposit was not applied; however, no blister was observed, to which the fiber deposit was applied.

### Example 3 (alkali cleansing treatment)

As a result of applying the sheet formulation as described in Preparation Example 1 hereafter to the cheek which was pretreated with an alkali cleansing agent followed by enzyme mask treatment, an excellent improvement tendency was observed. The alkali cleansing treatment was performed by an expert who was allowed to do so, and the sheet formulation was also applied by the expert.

### <Preparation Example 1: Sheet formulation>

Each component shown in Table 1 was weighed and the mixture was stirred with a propellant mixer at room temperature for 12 hours to manufacture a composition for forming a fiber deposit. The composition for forming a fiber deposit was used with an electrostatic method pursuant to the description of JP 2020-90097 to form a film and the film was trimmed to a size covering the area under the eyes and outer corner to prepare a sheet composed of a fiber deposit. The sheet has a fiber thickness (circle equivalent diameter) of 700 nm, basis weight of 1.3 g/m² and the fiber amount of 1.08 g/m².

| Component (% by mass) | | Preparation Example 1 |
|---|---|---|
| c2 | polyvinyl butyral *1 | 12.0 |
| c4 | distearyldimonium chloride *2 | 0.5 |
| c3 | 1,3-butylene glycol | 2.0 |
| c1 | aqueous ethanol solution (99.5% by mass) | 85.5 |
| Total (% by mass) | | 100.0 |

The symbols in Table 1 shows as follows.
*1 S-Lec B BM-1 (Sekisui Chemical Co., Ltd.)
*2 Varisoft TA100 (Evonik Japan Co., Ltd.)

### Example 4 Enzyme mask treatment

To the cheek which was pretreated with an enzyme mask treatment, the sheet composed of nanofibers prepared by electrostatic spraying in the same manner as Example 1 was applied. As a result, an excellent improvement tendency was observed. The enzyme mask treatment was performed by an expert who was allowed to do so.

### Example 5 Laser treatment

To the left cheek which was pretreated with a laser treatment, the fiber deposite composed of nanofibers was applied by electrostatic spraying in the same manner as Example 1. The sheet introduced in Example 3 was applied to the surrounding of the eyes. As a result, it was found that the left cheek was less dried and less stimulated than the right cheek. The laser treatment was performed by an expert who was allowed to do so, and fiber deposit by way of an electrostatic spraying and the sheet were applied by the panelist by herself.

### Example 6 acne treatment

To the jaw lines and arms which were pretreated with acne treatment, the sheet composed of nanofibers prepared by electrostatic spraying in the same manner as Example 1 was applied. The sheet introduced in Example 3 was applied to the surrounding of the mouth. As a result, an excellent improvement tendency was observed. The acne treatment was performed by an expert who was allowed to do so, and fiber deposit by way of electrostatic spraying and the sheet were applied by the panelist by herself.

### Example 7 Microneedling

Microneedling treatment was performed on cheeks, area under the eyes, jaw, and forehead, and then a fiber deposit composed of nanofibers was applied by electrostatic spraying. The sheet introduced in Example 3 was applied to the surrounding of the eyes and the surrounding of the mouth. As a result, an excellent improvement tendency was observed. The microneedling treatment was performed by a nurse, and fiber deposit by way of electrostatic spraying and the sheet were applied by the panelist by herself.

### Example 8 Wax treatment

Eyebrows were removed with a wax. The sheet introduced in Example 3 was applied. As a result, an excellent improvement tendency was observed. The wax treatment was performed by an expert who was allowed to do so. The sheet was also applied by the expert.

### Reference Signs List

- 10: Electrostatic spray device
- 11: Low-voltage power supply
- 12: High-voltage power supply
- 13: Auxiliary electric circuit
- 14: Pump mechanism
- 15: Container
- 16: Nozzle
- 17: Pipeline
- 18: Flexible pipeline
- 19: Current limiting resistance
- 20: Housing

## Claims

1. Composition X for use in a method for alleviating inflammation, redness and/or swelling of skin in a subject who has undergone a cosmetic or dermatological treatment,
wherein composition X comprises the following components (a) and (b):
a) one or more volatile substances selected from the group consisting of water, an alcohol, and a ketone; and
b) a polymer having an ability to form a film containing a fiber deposit; and
the method for alleviating inflammation, redness or swelling of skin comprises performing the following step A) and then step B):
A) physically or chemically treating an epidermis of the skin; and
B) forming a film containing a fiber deposit on the skin which has been subjected to the step A) with the composition X, wherein the step of forming a film containing a fiber deposit is performed by:
(B1) electrostatically spraying the composition X on the skin or
(B2) obtaining a film from the composition X by electrospinning or meltblowing on a surface, wherein the film is composed of a fiber deposit having an average fiber diameter of from 0.01 to 7 µm, and attaching said film to the skin.

2. The composition X for use according to claim 1, wherein in step (B1), the fibers contained in the film have an average fiber diameter of 0.01 µm or more and 7 µm or less.

3. The composition X for use according to claim 1 or 2, wherein the method further comprises a step C), before or after the step B),
C) applying a composition Y comprising one or more components selected from the group consisting of an oil agents and a polyol.

4. The composition X for use according to claim 3, wherein the composition Y used in the step C) further comprises an adhesive polymer.

5. The composition X for use according to any one of claims 1 to 4, wherein the polymer having an ability to form a film containing a fiber deposit (b) is a water-insoluble fiber-forming polymer.

6. The composition X for use according to claim 5, wherein the water-insoluble fiber-forming polymer is polyvinyl butyral resin or polyurethane resin.

7. Composition Z for use in a method for alleviating inflammation, redness and/or swelling of skin in a subject who has undergone a cosmetic or dermatological treatment,
wherein composition Z comprises the following components (c) and (d):
(c) one or more selected from the group consisting of an oil agent and a polyol which are in a liquid state at 20°C; and
(d) a fiber having an average fiber diameter ranging from 0.01 to 7 µm and an aspect ratio (average fiber length/average fiber diameter) ranging from 10 to 1,000; and
the method for alleviating inflammation, redness or swelling of skin comprises performing the following step A) and then step B):
A) physically or chemically treating an epidermis of the skin, wherein the treatment results in inflammation, redness or swelling of the skin; and
B) forming a film containing a fiber deposit on the skin which has been subjected to the step A) by applying the composition Z to the skin.

8. The composition X or Z for use according to any one of claims 1 to 7, wherein the step A) is physically or chemically treating one or more layers selected from the group consisting of a horny layer, a granular layer, a spinous layer, and a basal layer of the skin.

9. The composition X or Z for use according to any one of claims 1 to 8, wherein the step A) is a skin epidermis treatment selected from the group consisting of chemical peeling, alkali cleansing treatment, depilatory waxing, dermabrasion, microdermabrasion, microneedling, radiofrequency microneedling, laser treatment, IPL treatment, cryotherapy, tattoo treatment, tattoo removal, acne treatment, LED treatment, enzyme mask treatment and plasma fibroblast.

10. The composition Z for use according to any one of claims 7 to 9, wherein a polymer constituting the (d) fiber is a fiber-forming polymer which is water-insoluble.

## Patentansprüche

1. Zusammensetzung X zur Verwendung in einem Verfahren zur Linderung von Entzündung, Rötung und/oder Schwellung der Haut bei einem Subjekt, das sich einer kosmetischen oder dermatologischen Behandlung unterzogen hat,
wobei Zusammensetzung X die folgenden Komponenten (a) und (b) umfasst:
a) eine oder mehrere flüchtige Substanzen, ausgewählt aus der Gruppe bestehend aus Wasser, einem Alkohol und einem Keton; und
b) ein Polymer mit einer Fähigkeit zur Bildung eines Films, der eine Faserablagerung enthält; und
das Verfahren zur Linderung von Entzündung, Rötung oder Schwellung der Haut das Durchführen des folgenden Schritts A) und dann Schritts B) umfasst:
A) physikalisches oder chemisches Behandeln einer Epidermis der Haut; und
B) Bilden eines Films, der eine Faserablagerung enthält, auf der Haut, die dem Schritt A) unterzogen worden ist, mit der Zusammensetzung X, wobei der Schritt des Bildens eines Films, der eine Faserablagerung enthält, durchgeführt wird durch:
(B1) elektrostatisches Aufsprühen der Zusammensetzung X auf die Haut oder
(B2) Erhalten eines Films aus der Zusammensetzung X durch Elektrospinnen oder Schmelzblasen auf einer Oberfläche, wobei der Film aus einer Faserablagerung mit einem durchschnittlichen Faserdurchmesser von 0,01 bis 7 µm zusammengesetzt ist, und Anbringen des Films an der Haut.

2. Zusammensetzung X zur Verwendung nach Anspruch 1, wobei in Schritt (B1) die in dem Film enthaltenen Fasern einen durchschnittlichen Faserdurchmesser von 0,01 µm oder mehr und 7 µm oder weniger aufweisen.

3. Zusammensetzung X zur Verwendung nach Anspruch 1 oder 2, wobei das Verfahren, vor oder nach dem Schritt B), weiter einen Schritt C), umfasst,
C) Auftragen einer Zusammensetzung Y, die eine oder mehrere Komponenten umfasst, ausgewählt aus der Gruppe bestehend aus einem Ölmittel und einem Polyol.

4. Zusammensetzung X zur Verwendung nach Anspruch 3, wobei die in Schritt C) verwendete Zusammensetzung Y weiter ein Haftpolymer umfasst.

5. Zusammensetzung X zur Verwendung nach einem der Ansprüche 1 bis 4, wobei das Polymer mit einer Fähigkeit zur Bildung eines Films, der eine Faserablagerung (b), enthält, ein wasserunlösliches faserbildendes Polymer ist.

6. Zusammensetzung X zur Verwendung nach Anspruch 5, wobei das wasserunlösliche faserbildende Polymer Polyvinylbutyralharz oder Polyurethanharz ist.

7. Zusammensetzung Z zur Verwendung in einem Verfahren zur Linderung von Entzündung, Rötung und/oder Schwellung der Haut bei einem Subjekt, das sich einer kosmetischen oder dermatologischen Behandlung unterzogen hat,
wobei Zusammensetzung Z die folgenden Komponenten (c) und (d) umfasst:
(c) eines oder mehrere, ausgewählt aus der Gruppe bestehend aus einem Ölmittel und einem Polyol, die sich bei 20 °C in einem flüssigen Zustand befinden; und
(d) eine Faser mit einem durchschnittlichen Faserdurchmesser im Bereich von 0,01 bis 7 µm und einem Aspektverhältnis (durchschnittliche Faserlänge/durchschnittlicher Faserdurchmesser) im Bereich von 10 bis 1.000;
und
wobei das Verfahren zur Linderung von Entzündung, Rötung oder Schwellung der Haut das Durchführen des folgenden Schritts A) und dann des Schritts B) umfasst:
A) physikalisches oder chemisches Behandeln einer Epidermis der Haut, wobei die Behandlung zur Entzündung, Rötung oder Schwellung der Haut führt; und
B) Bilden eines Films, der eine Faserablagerung enthält, auf der Haut, die dem Schritt A) unterzogen worden ist, durch Auftragen der Zusammensetzung Z auf die Haut.

8. Zusammensetzung X oder Z zur Verwendung nach einem der Ansprüche 1 bis 7, wobei der Schritt A) das physikalische oder chemische Behandeln einer oder mehrerer Schichten ist, ausgewählt aus der Gruppe bestehend aus einer Hornschicht, einer Körnerschicht, einer Stachelschicht und einer Basalschicht der Haut.

9. Zusammensetzung X oder Z zur Verwendung nach einem der Ansprüche 1 bis 8, wobei der Schritt A) eine Hautepidermis-Behandlung ist, ausgewählt aus der Gruppe bestehend aus chemischem Peeling, alkalischer Reinigungsbehandlung, Enthaarungswachsen, Dermabrasion, Mikrodermabrasion, Mikronadelung, Hochfrequenz-Mikronadelung, Laserbehandlung, IPL-Behandlung, Kryotherapie, Tätowierungsbehandlung, Tätowierungsentfernung, Aknebehandlung, LED-Behandlung, Enzymmaskenbehandlung und Plasma-Fibroblast.

10. Zusammensetzung Z zur Verwendung nach einem der Ansprüche 7 bis 9, wobei ein Polymer, das die (d) Faser bildet, ein faserbildendes Polymer ist, welches wasserunlöslich ist.

## Revendications

1. Composition X pour une utilisation dans un procédé de soulagement d'une inflammation, d'une rougeur et/ou d'un gonflement de la peau d'un sujet qui a subi un traitement cosmétique ou dermatologique,
dans laquelle la composition X comprend les composants (a) et (b) suivants :
a) une ou plusieurs substances volatiles sélectionnées parmi le groupe consistant en l'eau, un alcool et une cétone ; et
b) un polymère présentant la capacité de former un film contenant un dépôt de fibres ; et le procédé de soulagement d'une inflammation, d'une rougeur ou d'un gonflement de la peau comprend la réalisation de l'étape A) et ensuite de l'étape B) suivantes :
A) le traitement physique ou chimique d'un épiderme de la peau ; et
B) la formation d'un film contenant un dépôt de fibres sur la peau qui a été soumise à l'étape A) avec la composition X, dans laquelle l'étape de formation d'un film contenant un dépôt de fibres est réalisée par :
(B1) la pulvérisation électrostatique de la composition X sur la peau ou
(B2) l'obtention d'un film à partir de la composition X par électrofilage ou soufflage à l'état fondu sur une surface, dans laquelle le film est composé d'un dépôt de fibre présentant un diamètre moyen de fibre de 0,01 à 7 µm, et la fixation dudit film à la peau.

2. Composition X pour une utilisation selon la revendication 1, dans laquelle à l'étape (B1), les fibres contenues dans le film présentent un diamètre moyen de fibre de 0,01 µm ou plus et de 7 µm ou moins.

3. Composition X pour une utilisation selon la revendication 1 ou la revendication 2, dans laquelle le procédé comprend en outre une étape C), avant ou après l'étape B),
C) l'application d'une composition Y comprenant un ou plusieurs composants sélectionnés dans le groupe consistant en un agent huileux et un polyol.

4. Composition X pour une utilisation selon la revendication 3, dans laquelle la composition Y utilisée à l'étape C) comprend en outre un polymère adhésif.

5. Composition X pour une utilisation selon l'une quelconque des revendications 1 à 4, dans laquelle le polymère présentant la capacité de former un film contenant un dépôt de fibres (b) est un polymère formant fibre insoluble dans l'eau.

6. Composition X pour une utilisation selon la revendication 5, dans laquelle le polymère formant fibre insoluble dans l'eau est une résine de polyvinylbutyral ou une résine de polyuréthane.

7. Composition Z pour une utilisation dans un procédé de soulagement d'une inflammation, d'une rougeur et/ou d'un gonflement de la peau d'un sujet qui a subi un traitement cosmétique ou dermatologique,
dans laquelle la composition Z comprend les composants (c) et (d) suivants :
(c) un ou plusieurs sélectionnés dans le groupe consistant en un agent huileux et un polyol qui sont à l'état liquide à 20 °C ; et
(d) une fibre présentant un diamètre moyen de fibre allant de 0,01 à 7 µm et un rapport d'aspect (longueur moyenne de fibre/diamètre moyen de fibre) allant de 10 à 1 000 ; et
le procédé de soulagement d'une inflammation, d'une rougeur ou d'un gonflement de la peau comprend la réalisation de l'étape A) et ensuite de l'étape B) suivantes :
A) le traitement physique ou chimique d'un épiderme de la peau, dans laquelle le traitement entraîne une inflammation, une rougeur ou un gonflement de la peau ; et
B) la formation d'un film contenant un dépôt de fibres sur la peau qui a été soumise à l'étape A) par l'application de la composition Z sur la peau.

8. Composition X ou Z pour une utilisation selon l'une quelconque des revendications 1 à 7, dans laquelle l'étape A) est un traitement physique ou chimique d'une ou plusieurs couches sélectionnées dans le groupe consistant en une couche cornée, une couche granuleuse, une couche épineuse et une couche basale de la peau.

9. Composition X ou Z pour une utilisation selon l'une quelconque des revendications 1 à 8, dans laquelle l'étape A) est un traitement de l'épiderme cutané sélectionné dans le groupe consistant en gommage chimique, traitement nettoyant alcalin, cirage dépilatoire, dermabrasion, microdermabrasion, microneedling, microneedling par radiofréquence, traitement au laser, traitement IPL, cryothérapie, traitement de tatouage, retrait de tatouage, traitement de l'acné, traitement par DEL, traitement par masque enzymatique et plasma fibroblast.

10. Composition Z pour une utilisation selon l'une quelconque des revendications 7 à 9, dans laquelle un polymère constituant la (d) fibre est un polymère formant fibre qui est insoluble dans l'eau.
